# EUROPÄISCHE PATENTANMELDUNG

(11) **EP 1 950 268 A1**
(43) Veröffentlichungstag der Anmeldung: **30.07.2008**
(21) Anmeldenummer: 08000687.7
(22) Anmeldetag: 15.01.2008
(51) Int. Cl.: C10G 2/00, C10G 9/00, C10G 11/00, C10G 35/00, C10G 57/00, C10G 63/04, C07C 4/20

(54) **Verfahren zur Dampf-Dealkylierung unter Einbindung einer Wassergas-Shift-Reaktion**

(30) Priorität: 26.01.2007 DE 102007004078
(71) Anmelder: Linde Aktiengesellschaft, 80807 München (DE)
(72) Erfinder: Göke, Volker, 82515 Wolfrathausen (DE); Neuendorf, Stephanie, 82069 Hohenschäftlarn (DE); Ponceau, Marianne, 80634 München (DE); Schödel, Nicole, 81477 München (DE); Wenning, Ulrike, 82049 Pullach (DE)

(57) **Zusammenfassung**

Die Erfindung beschreibt ein Verfahren zur Behandlung der gasförmigen Reaktionsprodukte der Dampf-Dealkylierung einer Kohlenwasserstofffraktion mit mindestens sechs Kohlenstoffatomen (C₆₊-Fraktion) und/oder einer Kohlenwasserstofffraktion mit mindestens sieben Kohlenstoffatomen (C₇₊-Fraktion) in einer Anlage zur Erzeugung von Kohlenwasserstoffen aus kohlenwasserstoffhaltigem Einsatz, wobei als gasförmige Reaktionsprodukte der Dampf-Dealkylierung vorwiegend Wasserstoff, Kohlenmonoxid, Kohlendioxid und Methan entstehen. Die jeweilige Kohlenwasserstofffraktion (1) wird über einen Wärmetauscher (2) als Einsatzstoff in ein Verfahren zur Dampf-Dealkylierung (3) geführt. Die Reaktionsprodukte der Dampf-Dealkylierung (4) werden über den Wärmetauscher (2), der gleichzeitig die Kohlenwasserstofffraktion (1) vorwärmt, und einen weiteren Kühler (nicht dargestellt) abgekühlt und zu einem 3-Phasenseparator (5) geführt. Dort werden die gasförmigen Reaktionsprodukte (6) von den Kohlenwasserstoffen (7) und Wasser (8) getrennt. Aus den Kohlenwasserstoffen (7) wird das gewünschte Wertprodukt Benzol (9) abgetrennt, während die restlichen Kohlenwasserstoffe (10) ebenso wie das Wasser (8) erneut in den Einsatz zur Dampf-Dealkylierung gefahren werden. Zur Abtrennung von Kohlendioxid wird eine Flüssig-Gas-Extraktion mit einer ionischen Flüssigkeit als Extraktionsmittel verwendet. Die ionische Flüssigkeit wird mit Kohlendioxid beladen (11) und anschließend zur Regeneration (12) geführt. Dort wird die ionische Flüssigkeit erwärmt, so dass Kohlendioxid aus dem Extraktionsmittel entweicht und als Produkt aus der Anlage geführt wird (13). Die regenerierte Flüssigkeit (14) wird zurück zur Beladung geführt. Die nunmehr kohlendioxidfreien, überwiegend Kohlenmonoxid, Wasserstoff und Methan enthaltenden gasförmigen Reaktionsprodukte (15) werden zusammen mit Wasser (16) als Einsatzstoff in ein Verfahren zur Durchführung einer Wassergas-Shift-Reaktion (17) geführt. Das in den gasförmigen Reaktionsprodukten vorhandene Kohlenmonoxid reagiert hier mit Wasser zu Kohlendioxid und Wasserstoff. Die Reaktionsprodukte der Wassergas-Shift-Reaktion (18) bestehen somit überwiegend aus Kohlendioxid, Methan und einen deutlich erhöhten Anteil an Wasserstoff.

## Beschreibung

Die Erfindung betrifft Verfahren zur Behandlung der gasförmigen Reaktionsprodukte der Dampf-Dealkylierung einer Kohlenwasserstofffraktion mit mindestens sechs Kohlenstoffatomen (C₆₊-Fraktion) und/oder einer Kohlenwasserstofffraktion mit mindestens sieben Kohlenstoffatomen (C₇₊-Fraktion) in einer Anlage zur Erzeugung von Kohlenwasserstoffen aus kohlenwasserstoffhaltigem Einsatz, wobei als gasförmige Reaktionsprodukte der Dampf-Dealkylierung vorwiegend Wasserstoff, Kohlenmonoxid, Kohlendioxid und Methan entstehen.

In einer Anlage zur Erzeugung von Kohlenwasserstoffen aus kohlenwasserstoffhaltigem Einsatz entsteht ein Gemisch aus Kohlenwasserstoffen mit einer unterschiedlichen Anzahl von Kohlenstoffatomen und unterschiedlichen molekularen Konfigurationen. Dieses Gemisch wird in der Anlage zur Erzeugung von Kohlenwasserstoffen aus kohlenwasserstoffhaltigem Einsatz in die einzelnen Kohlenwasserstoffprodukte aufgetrennt.

Abhängig von der jeweiligen Trennsequenz und der Art der Anlage zur Erzeugung von Kohlenwasserstoffen aus kohlenwasserstoffhaltigem Einsatz entstehen eine Kohlenwasserstofffraktion mit mindestens sechs Kohlenstoffatomen (C₆₊-Fraktion) und/oder eine Kohlenwasserstofffraktion mit mindestens sieben Kohlenstoffatomen (C₇₊-Fraktion). Diese Fraktionen enthalten als wirtschaftlich verwertbares Produkt Aromaten, welche als Ausgangsstoff für die Synthese zahlreicher Kunststoffe und zur Erhöhung der Klopffestigkeit von Benzin Verwendung finden.

Um an die wirtschaftlich verwertbaren Produkte der C₆₊-Fraktion, vor allem Benzol, zu gelangen und die Ausbeute möglichst maximal zu gestalten, wird nach dem Stand der Technik folgendes Verfahren angewendet. Die C₆₊-Fraktion wird in eine Fraktion überwiegend bestehend aus Kohlenwasserstoffen mit sechs Kohlenstoffatomen (C₆-Fraktion) und eine C₇₊-Fraktion getrennt. Aus der C₆-Fraktion kann direkt das wirtschaftlich verwertbare Produkt Benzol abgetrennt werden. Aus der C₇₊-Fraktion werden mittels einer Flüssig-Flüssig Extraktion die linearen Kohlenwasserstoffe abgetrennt und als so genanntes Raffinat weiterverarbeitet. Die von den linearen Kohlenwasserstoffen befreite C₇₊-Fraktion enthält nunmehr hauptsächlich Aromaten mit sieben bis acht Kohlenstoffatomen und wird in eine Fraktion überwiegend bestehend aus Kohlenwasserstoffen mit sieben Kohlenstoffatomen (hauptsächlich Toluol) und in eine Fraktion überwiegend bestehend aus Kohlenwasserstoffen mit acht Kohlenstoffatomen (hauptsächlich Xylol) getrennt. Die Fraktion überwiegend bestehend aus Kohlenwasserstoffen mit acht Kohlenstoffatomen wird als Einsatzstoff in ein Verfahren zur para-Xylol Gewinnung geführt. Die Fraktion überwiegend bestehend aus Kohlenwasserstoffen mit sieben Kohlenstoffatomen wird als Einsatz in ein Verfahren zur Hydro-Dealkylierung geführt wird.

Ein derartiges Verfahren zur Hydro-Dealkylierung ist zum Beispiel in W02005071045 beschrieben. Die Kohlenwasserstoffe werden in Anwesenheit eines Katalysators bei einer Temperatur von 400°C bis 650°C und einem Druck zwischen 20 bar und 40 bar mit Wasserstoff kontaktiert, wobei der Wasserstoff in einem molaren Überschuss vom drei- bis sechsfachen der Kohlenwasserstoffe vorliegt. Unter diesen Bedingungen werden die Alkylgruppen von den jeweiligen alkylierten Aromaten (wie zum Beispiel Toluol) abgespalten, so dass sich Benzol und die jeweiligen Alkane (zum Beispiel Methan) bilden.

Der Verbrauch von Wasserstoff bei der Hydro-Dealkylierung der Kohlenwasserstoffe wirkt sich negativ auf die Wirtschaftlichkeit dieses Verfahrens nach dem Stand der Technik zur Benzolgewinnung aus.

In einem alternativen Verfahren werden die C₆₊-Fraktion und/oder die C₇₊-Fraktion einer Dampf-Dealkylierung unterzogen (siehe zum Beispiel eingereichte Patentschriften DE102006058528.3 oder DE102006058529.1). Bei einer Dampf-Dealkylierung der C₆₊-Fraktion und/oder C₇₊-Fraktion entstehen hauptsächlich die beiden verwertbaren Produktstoffe Benzol und Wasserstoff neben Reaktionsprodukten wie Kohlenmonoxid und Kohlendioxid. Als Einsatzstoff der Dampf-Dealkylierung wird somit lediglich kostengünstiger Wasserdampf bei gleichzeitiger Produktion des gewünschten Wertproduktes Benzol und des wertvollen Nebenproduktes Wasserstoff benötigt.

In diesem Verfahren nach dem Stand der Technik reagieren die Kohlenwasserstoffe aus der jeweiligen Kohlenwasserstofffraktion mit Wasserdampf in der Gasphase an einem festen Katalysator. Dabei werden die Kohlenwasserstoffe und der Wasserdampf in einer Vorrichtung, zum Beispiel Rohre, an dem festen Katalysator vorbeigeführt, wobei sich der Katalysator im Inneren der Vorrichtung, zum Beispiel im Rohrinneren, befindet. Die für die Dealkylierung nötige Reaktionswärme wird der Vorrichtung von außen zugeführt, zum Beispiel durch Verbrennung der gasförmigen Reaktionsnebenprodukte Kohlenmonoxid und Methan mit Luft. Nach dem Stand der Technik entspricht die Temperatur der Einsatzstoffe der Temperatur der Reaktionsprodukte und liegt im Bereich von 400°C bis 600°C. Das Katalysatormaterial im Inneren der Vorrichtung besteht im Wesentlichen aus einem porösen Trägermaterial wie γ-Al₂O₃, MgAl Spinell und/oder Cr₂O₃, und einer an der Oberfläche des Trägermaterials befindlichen Aktivkomponente, wie Rh mit 0.1-1.0 Gew% Beladung und/oder Pd mit 0.2-2.0 Gew% Beladung. Aus den gasförmigen Reaktionsprodukten der Dampf-Dealkylierung wird über eine Druckwechseladsorption mit vorheriger Verdichtung der wirtschaftlich verwertbare Wasserstoff von den Reaktionsnebenprodukten wie Kohlenmonoxid, Kohlendioxid und Methan getrennt.

Nach dem Stand der Technik wird von den gasförmigen Reaktionsprodukten nur das Wertprodukt Wasserstoff abgetrennt, während der Rest der gasförmigen Reaktionsprodukte als Brenngas Verwendung findet.

Der Erfindung liegt daher die Aufgabe zugrunde, die Behandlung der gasförmigen Reaktionsprodukte der Dampf-Dealkylierung derart zu optimieren, dass der Anteil des Wertproduktes Wasserstoff erhöht wird.

Die vorliegende Aufgabe wird dadurch gelöst, dass die gasförmigen Reaktionsprodukte ganz oder zumindest teilweise als Einsatzstoff in ein Verfahren zur Durchführung einer Wassergas-Shift-Reaktion geführt werden, wobei in der Wassergas-Shift-Reaktion aus Kohlenmonoxid und Wasser die Reaktionsprodukte Kohlendioxid und Wasserstoff gebildet werden. Durch das erfindungsgemäße Verfahren wird nicht nur bei der Dampf-Dealkylierung sondern auch zusätzlich in der Wassergas-Shift-Reaktion das Wertprodukt Wasserstoff gebildet. Dadurch erhöht sich die Gesamtmenge des Wertproduktes Wasserstoff gegenüber dem Stand der Technik deutlich.

In einer bevorzugten Ausgestaltung der Erfindung wird Kohlendioxid aus den gasförmigen Reaktionsprodukten entfernt, bevor die gasförmigen Reaktionsprodukte als Einsatzstoff in ein Verfahren zur Durchführung einer Wassergas-Shift-Reaktion geführt werden. Durch die Entfernung von Kohlendioxid aus dem Einsatzstoff der Wassergas-Shift-Reaktion und damit durch die Entfernung eines Reaktionsproduktes der Wassergas-Shift-Reaktion verschiebt sich das Reaktionsgleichgewicht der Wassergas-Shift-Reaktion zugunsten der Reaktionsprodukte und der Anteil an gebildeten Wasserstoff erhöht sich.

In einer anderen Ausgestaltung der Erfindung wird Kohlenmonoxid aus den gasförmigen Reaktionsprodukten entfernt und als Einsatzstoff in ein Verfahren zur Wassergas-Shift-Reaktion geführt. Wird Kohlenmonoxid von den gasförmigen Reaktionsprodukten abgetrennt und nur dieses als Einsatzstoff in das Verfahren zur Durchführung der Wassergas-Shift-Reaktion geführt, muss nur ein wesentlich kleinerer Gasstrom in der Wassergas-Shift-Reaktion verarbeitet werden. Zusätzlich erhöht sich der Partialdruck des Kohlenmonoxids und das Reaktionsgleichgewicht der Wassergas-Shift-Reaktion wird zugunsten der Reaktionsprodukte verschoben und die Wasserstoffausbeute erhöht.

In einer weiteren Ausgestaltung der Erfindung wird Wasserstoff aus den gasförmigen Reaktionsprodukten entfernt, bevor die gasförmigen Reaktionsprodukte als Einsatzstoff in ein Verfahren zur Durchführung einer Wassergas-Shift-Reaktion geführt werden. Als ebenso vorteilhaft erweist sich die Entfernung von Wasserstoff und Kohlendioxid aus den gasförmigen Reaktionsprodukten, bevor die gasförmigen Reaktionsprodukte als Einsatzstoff in ein Verfahren zur Durchführung einer Wassergas-Shift-Reaktion geführt werden. Sowohl Wasserstoff als auch Kohlendioxid können als Wertprodukte gewonnen und verarbeitet werden. Durch die Entfernung der beiden Komponenten vor der Wassergas-Shift-Reaktion wird die dort zur verarbeitende Gasmenge deutlich reduziert.

Vorteilhafterweise wird der Wasserstoff aus den Reaktionsprodukten der Wassergas-Shift-Reaktion abgetrennt und als Einsatzstoff in ein Verfahren unter Wasserstoffverbrauch innerhalb der Anlage, als Einsatzstoff in den Eingangsbereich der Dampf-Dealkylierung und/oder als Reinprodukt zur Verwertung aus der Anlage geführt. Das Wertprodukt Wasserstoff kann beispielsweise mittels eines Verfahrens zur Druckwechseladsorption aus den Reaktionsprodukten der Wassergas-Shift-Reaktion abgetrennt und beliebig verwertet werden, wobei entweder eine Verwertung in den zahlreichen Wasserstoff verbrauchenden Verfahren der Anlage oder eine Verwertung außerhalb der Anlage wie zum Beispiel in einer Brennstoffzelle möglich ist.

Zweckmäßigerweise wird Kohlendioxid aus den Reaktionsprodukten der Wassergas-Shift-Reaktion abgetrennt und als Reinprodukt zur Verwertung aus der Anlage geführt. Kohlendioxid kann als Reinprodukt in vielfältigen Anwendungen außerhalb der Anlage dienen.

Als ebenso zweckmäßig erweist sich die Abtrennung von Methan und anderer leichter Alkane aus den Reaktionsprodukten der Wassergas-Shift-Reaktion und deren Verwendung als Einsatzstoff einer Verbrennung, die zum Beispiel zur Erzeugung der bei der Dampf-Dealkylierung nötigen Wärme dient.

Mit der vorliegenden Erfindung gelingt es insbesondere, den Anteil des bei der Dampf-Dealkylierung gewonnenen Wertproduktes Wasserstoff zu steigern.

Im Folgenden soll die Erfindung anhand der grafischen Darstellung eines Ausführungsbeispiels der Erfindung näher erläutert werden.

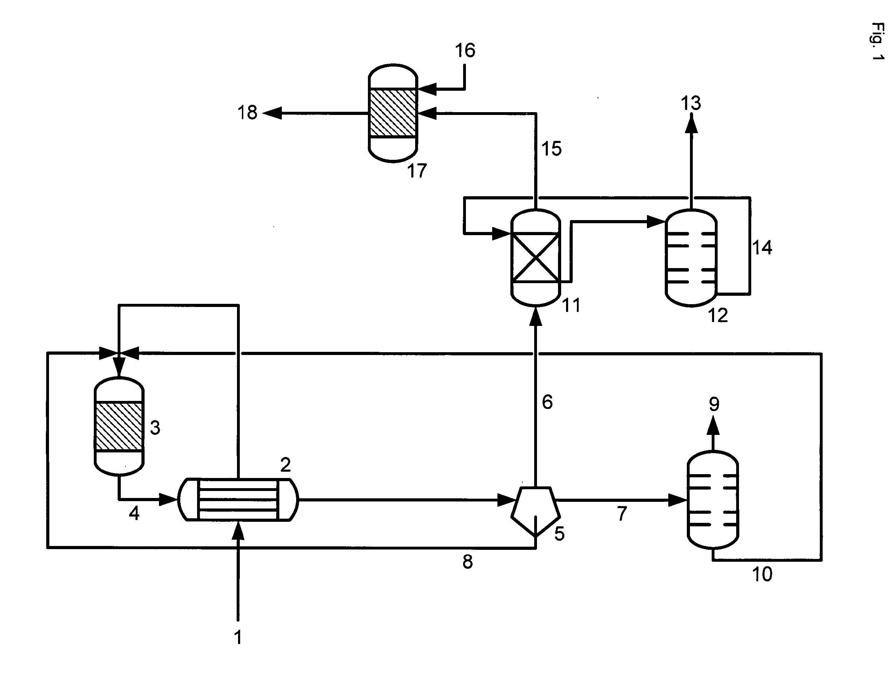

Figur 1 zeigt das Verfahrensschema einer Ausgestaltung der Erfindung. Die jeweilige Kohlenwasserstofffraktion (C₆₊-Fraktion oder C₇₊-Fraktion) (1) wird über einen Wärmetauscher (2) als Einsatzstoff in ein Verfahren zur Dampf-Dealkylierung (3) geführt. Die Reaktionsprodukte der Dampf-Dealkylierung (4) werden über den Wärmetauscher (2), der gleichzeitig die Kohlenwasserstofffraktion (1) vorwärmt, und einen weiteren Kühler (nicht dargestellt) abgekühlt und zu einem 3-Phasenseparator (5) geführt. Dort werden die gasförmigen Reaktionsprodukte (6) von den Kohlenwasserstoffen (7) und Wasser (8) getrennt. Aus den Kohlenwasserstoffen (7) wird das gewünschte Wertprodukt Benzol (9) abgetrennt, während die restlichen Kohlenwasserstoffe (10) ebenso wie das Wasser (8) erneut in den Einsatz zur Dampf-Dealkylierung gefahren werden. Die gasförmigen Reaktionsprodukte werden in ein Verfahren zur Abtrennung von Kohlendioxid geführt. Zur Abtrennung von Kohlendioxid wird in dieser Ausgestaltung der Erfindung eine Flüssig-Gas-Extraktion mit einer ionischen Flüssigkeit als Extraktionsmittel verwendet. Die ionische Flüssigkeit wird mit Kohlendioxid beladen (11) und anschließend zur Regeneration (12) geführt. Dort wird die ionische Flüssigkeit erwärmt, so dass Kohlendioxid aus dem Extraktionsmittel entweicht und als Produkt aus der Anlage geführt wird (13). Die regenerierte Flüssigkeit (14) wird zurück zur Beladung geführt. Die nunmehr kohlendioxidfreien, überwiegend Kohlenmonoxid, Wasserstoff und Methan enthaltenden gasförmigen Reaktionsprodukte (15) werden zusammen mit Wasser (16) als Einsatzstoff in ein Verfahren zur Durchführung einer Wassergas-Shift-Reaktion (17) geführt. Das in den gasförmigen Reaktionsprodukten vorhandene Kohlenmonoxid reagiert hier mit Wasser zu Kohlendioxid und Wasserstoff. Die Reaktionsprodukte der Wassergas-Shift-Reaktion (18) bestehen somit überwiegend aus Kohlendioxid, nicht umgesetztem Methan und einem erhöhten Anteil an Wasserstoff. Der Wasserstoff kann in einem späteren Verfahrensschritt zum Beispiel mittels eines Druckwechseladsorptionsverfahrens aus den Reaktionsprodukten der Wassergas-Shift-Reaktion gewonnen werden.

## Patentansprüche

1. Verfahren zur Behandlung der gasförmigen Reaktionsprodukte der Dampf-Dealkylierung einer Kohlenwasserstofffraktion mit mindestens sechs Kohlenstoffatomen (C₆₊-Fraktion) und/oder einer Kohlenwasserstofffraktion mit mindestens sieben Kohlenstoffatomen (C₇₊-Fraktion) in einer Anlage zur Erzeugung von Kohlenwasserstoffen aus kohlenwasserstoffhaltigem Einsatz, wobei als gasförmige Reaktionsprodukte der Dampf-Dealkylierung vorwiegend Wasserstoff, Kohlenmonoxid, Kohlendioxid und Methan entstehen, **dadurch gekennzeichnet, dass** die gasförmigen Reaktionsprodukte ganz oder zumindest teilweise als Einsatzstoff in ein Verfahren zur Durchführung einer Wassergas-Shift-Reaktion geführt werden, wobei in der Wassergas-Shift-Reaktion aus Kohlenmonoxid und Wasser die Reaktionsprodukte Kohlendioxid und Wasserstoff gebildet werden.

2. Verfahren nach Anspruch 1, **dadurch gekennzeichnet, dass** Kohlendioxid aus den gasförmigen Reaktionsprodukten entfernt wird, bevor die gasförmigen Reaktionsprodukte als Einsatzstoff in ein Verfahren zur Durchführung einer Wassergas-Shift-Reaktion geführt werden.

3. Verfahren nach Anspruch 1 oder 2, **dadurch gekennzeichnet, dass** Kohlenmonoxid aus den gasförmigen Reaktionsprodukten entfernt und als Einsatzstoff in ein Verfahren zur Wassergas-Shift-Reaktion geführt wird.

4. Verfahren nach einem der Ansprüche 1 bis 3, **dadurch gekennzeichnet, dass** Wasserstoff aus den gasförmigen Reaktionsprodukten entfernt wird, bevor die gasförmigen Reaktionsprodukte als Einsatzstoff in ein Verfahren zur Durchführung einer Wassergas-Shift-Reaktion geführt werden.

5. Verfahren nach einem der Ansprüche 1 bis 4, **dadurch gekennzeichnet, dass** Wasserstoff und Kohlendioxid aus den gasförmigen Reaktionsprodukten entfernt werden, bevor die gasförmigen Reaktionsprodukte als Einsatzstoff in ein Verfahren zur Durchführung einer Wassergas-Shift-Reaktion geführt werden.

6. Verfahren nach einem der Ansprüche 1 bis 5, **dadurch gekennzeichnet, dass** Wasserstoff aus den Reaktionsprodukten der Wassergas-Shift-Reaktion abgetrennt und als Einsatzstoff in ein Verfahren unter Wasserstoffverbrauch innerhalb der Anlage, als Einsatzstoff in den Eingangsbereich der Dampf-Dealkylierung und/oder als Reinprodukt zur Verwertung aus der Anlage geführt wird.

7. Verfahren nach einem der Ansprüche 1 bis 6, **dadurch gekennzeichnet, dass** Kohlendioxid aus den Reaktionsprodukten der Wassergas-Shift-Reaktion abgetrennt und als Reinprodukt zur Verwertung aus der Anlage geführt wird.

8. Verfahren nach einem der Ansprüche 1 bis 7, **dadurch gekennzeichnet, dass** Methan und anderer leichter Alkane aus den Reaktionsprodukten der Wassergas-Shift-Reaktion abgetrennt und als Einsatzstoff in eine Verbrennung geführt werden.
